# EUROPEAN PATENT APPLICATION

(11) **EP 4 516 399 A1**
(43) Date of publication of application: **05.03.2025**
(21) Application number: 23795515.8
(22) Date of filing: 27.04.2023
(51) Int. Cl.: B01J 38/00, B01J 38/12

(54) **CATALYTIC CRACKING CATALYST REGENERATION METHOD AND SYSTEM USING BIO-BASED GAS-PHASE FUEL**

(30) Priority: 29.04.2022 CN 202210476023; 29.04.2022 CN 202210476007; 29.04.2022 CN 202210474594; 29.04.2022 CN 202210476013
(71) Applicant: China Petroleum & Chemical Corporation, Beijing 100728 (CN); Sinopec Research Institute of Petroleum Processing Co., Ltd., Beijing 100083 (CN)
(72) Inventor: XU, Youhao, Beijing 100083 (CN); YANG, Wenjie, Beijing 100083 (CN); HE, Mingyuan, Beijing 100083 (CN); WANG, Xin, Beijing 100083 (CN)
(74) Representative: karo IP
(86) International application number: PCT/CN2023/091025
(87) International publication number: WO 2023/208083

(57) **Abstract**

Disclosed is a catalyst regeneration method suitable for a fluidized catalytic cracking unit comprising a catalytic cracking reactor and a catalyst regenerator, the regeneration method comprising the following steps: 1) providing a gaseous biomass-derived fuel containing hydrogen and/or methane; 2) directly feeding the gaseous fuel into the catalyst regenerator without separation and purification; 3) introducing an oxygen-containing gas into the catalyst regenerator, wherein the oxygen content of the oxygen-containing gas is 14-28% by volume; and 4) feeding the catalyst to be regenerated from the catalytic cracking reactor into the catalyst regenerator, where it contacts the gaseous fuel and the oxygen-containing gas for coke-burning and regeneration. The method of the present application introduces a gaseous biomass-derived fuel as energy supply in the catalyst regeneration process to replace fossil fuels, fundamentally changing the energy source of the catalytic cracking unit, significantly reducing the carbon emissions of the catalytic cracking unit, realizing the recycling of carbon elements, and supplying energy to other process units.

## Description

### Technical Field

The present application relates to the regeneration of a coke-containing catalytic cracking catalyst, and in particular to a method and system for regenerating a catalytic cracking catalyst using a gaseous bio-based fuel.

### Background Art

Nowadays, the development of the global refining industry faces many challenges such as new energy substitution and stricter requirements for energy conservation and emission reduction. Flexibly adjusting cracking production plans, reducing carbon dioxide emissions, and mitigating climate change have become the only way for the refining industry to transform its economic growth model and maintain sustainable development. It is urgent to achieve carbon peak in 2030 and carbon neutrality in 2060. The "14th Five-Year Plan" had formulated a carbon peak action plan, which clearly requires accelerating the promotion of green development. China's national carbon emission rights trading market has also been officially launched in 2021. Therefore, it is particularly important to effectively reduce carbon emissions in the process of petroleum refining and chemical production. The research on low-carbon catalytic cracking schemes to reduce oil and increase chemical production is an important task for refineries in the future. Carbon emissions in the heavy oil processing process are mainly flue gas emissions from catalytic cracking coke-burning, hydrogen production process, boilers and other equipment, and energy consumption in the processes. Among them, the catalytic cracking unit is the core equipment in the refinery. The carbon emissions caused by the coke-burning of the catalytic cracking regenerator account for 24-55% of the carbon emissions of the whole plant and nearly 1% of the total carbon dioxide emissions in the whole country. It is the focus of carbon emission reduction in the petrochemical industry.

CN 102 698 817 A discloses a catalytic cracking catalyst regeneration method, which adopts pure oxygen regeneration, couples a steam shift reaction zone after the flue gas energy recovery system, employs CO in the flue gas as a raw material for the steam shift reaction, to produce hydrogen, and further recycle and recover the carbon in the flue gas. However, this method only focuses on the treatment and recovery of flue gas, and the incomplete regeneration flue gas reduces the energy utilization rate. It also involves the separation and purification of raw materials and products in the steam shift reaction zone, which is costly; and does not change the energy source of the regeneration energy supply.

CN 113 877 397 A discloses an incomplete regeneration process for reducing carbon dioxide emissions. The process uses pure oxygen to incompletely regenerate the catalyst. In the obtained flue gas, the carbon monoxide is used as a chemical raw material, and the carbon dioxide is used for storage or oil recovery, thereby reducing carbon emissions. However, the process mainly involves the post-treatment process of flue gas, which is costly. It involves the separation of carbon monoxide, carbon dioxide, oxygen and other gases, and the separation process is complicated. Incomplete regeneration does not maximize the use of the chemical energy of deposited coke, and the storage of enriched carbon dioxide causes a waste of resources.

US 5 565 089 discloses a catalytic cracking catalyst regeneration process, which first uses air to burn the coke, then recovers the carbon dioxide in the flue gas, circulates it and gradually merges it into the oxygen-containing gas flow until the temperature in the regenerator is normal, and finally injects only oxygen and carbon dioxide to regenerate the catalyst. This process focuses on the gas intake system and flue gas treatment of the regeneration process, but the carbon dioxide generated by the energy supply still all comes from fossil energy.

The energy of the catalytic cracking unit comes from the coke-burning of the catalyst. When more low-carbon olefins and other chemicals are produced, more reaction heat is required due to the high gas yield. When the amount of coke burned is not enough to meet the energy consumption of the unit, the amount of coke generated is usually increased by recycling oil slurry, increasing the proportion of heavy oil in the feedstock oil, so as to increase the regeneration temperature, or the regeneration temperature is increased by injecting fuel oil for combustion. All these three methods can meet the heat balance of the reaction, but all will have a certain impact on the operation of the unit. And the supplementary energy all comes from fossil energy sources, which increases the carbon dioxide emissions from fossil energy sources and is not conducive to improving the utilization rate of petroleum resources. Optimizing the regeneration process can improve energy utilization efficiency, thereby reducing unit carbon dioxide emissions to a certain extent; recycling and recovering the emitted carbon dioxide can also reduce carbon dioxide emissions to a certain extent, but the cost is high and the process is relatively complicated. However, the above ideas do not fundamentally change the source of energy, and carbon dioxide still comes from fossil energy sources.

Therefore, it is necessary to develop a catalyst regeneration method that can fundamentally reduce carbon dioxide emissions from fossil energy sources, reduce carbon dioxide emissions while meeting the energy supply required by the unit, and achieve low-carbon development.

### Content of the invention

It is an object of the present application to provide a catalyst regeneration method and system suitable for a fluidized catalytic cracking unit, wherein the method and system introduce gaseous fuel derived from biomass into the catalyst regeneration system for combustion to provide energy, maintaining the thermal balance of the fluidized catalytic cracking unit, and thereby fundamentally reducing carbon dioxide emissions from fossil energy sources.

In order to achieve the above object, on the one hand, the present application provides a catalyst regeneration method suitable for a fluidized catalytic cracking unit comprising a catalytic cracking reactor and a catalyst regenerator, and the regeneration method comprises the following steps:
1) providing a gaseous biomass-derived fuel containing hydrogen and/or methane;
2) directly feeding the gaseous fuel into the catalyst regenerator without separation and purification;
3) introducing an oxygen-containing gas into the catalyst regenerator, wherein the oxygen content of the oxygen-containing gas is 14-28% by volume; and
4) feeding the catalyst to be regenerated from the catalytic cracking reactor into the catalyst regenerator, where it contacts with the gaseous fuel and the oxygen-containing gas for coke-burning and regeneration.

On the other hand, the present application provides a catalyst regeneration system suitable for a fluidized catalytic cracking unit, comprising a biomass processing unit and a catalyst regeneration unit, wherein:
the biomass processing unit is used to process biomass to obtain a gaseous fuel containing hydrogen and/or methane, and comprises a gaseous fuel generator and a gaseous fuel storage tank, wherein the gaseous fuel generator is preferably selected from a biomass gasifier, a biomass anaerobic fermentation tank or a combination thereof, and has a biomass inlet and a gaseous product outlet, wherein the gaseous fuel storage tank has an inlet and a gaseous fuel outlet, wherein the gaseous product outlet of the gaseous fuel generator is connected to the inlet of the gaseous fuel storage tank;
wherein the catalyst regeneration unit is used to regenerate the catalyst to be regenerated from the catalytic cracking reactor, and comprises a catalyst regenerator, wherein the catalyst regenerator has a catalyst to be regenerated inlet, an oxygen-containing gas inlet, a gaseous fuel inlet, a regeneration flue gas outlet, and a regenerated catalyst outlet, and
wherein the gaseous fuel outlet of the gaseous fuel storage tank is connected to the gaseous fuel inlet of the catalyst regenerator through a pipeline.

Comparing with the existing catalytic cracking catalyst regeneration methods and systems, the main advantages of the catalyst regeneration method and system of the present application are as follows:
(1) Biomass is cheap and readily available. Biomass energy is a renewable energy source, wherein the carbon thereof comes from the carbon dioxide captured by plants from the atmosphere, rather than fossil energy source. Using biomass as energy source can fundamentally change the source of energy supply for catalytic cracking units, reduce carbon dioxide emissions from fossil energy source, and achieve low-carbon development of refining.
(2) The gaseous fuel produced from biomass can be used directly without complex separation and purification, thus reducing the utilization cost;
(3) The gaseous fuel produced from biomass has a relatively high hydrogen content. The steam generated by the combustion of the hydrogen contained therein can be used to age the catalyst during the catalyst regeneration process to improve the selectivity of the target light olefin products. Alternatively, when the catalyst needs to have high activity, the adverse effect of steam on the catalyst activity can be weakened by optimizing the regeneration process.
(4) When gaseous fuel is used as the combustion medium, it is mixed more evenly with the catalyst to be generated, and the combustion and heat transfer processes are more stable.
(5) When oxygen-enriched regeneration is adopted, the carbon dioxide in the regeneration flue gas can be separated and captured at low cost, which is conducive to achieving negative carbon emissions;
(6) The excess heat generated by the regeneration system can be used to supply other units, allowing the catalytic cracking unit to gradually develop into the power center of an integrated refining and chemical enterprise.

Other features and advantages of the present application will be described in details in the subsequent specific embodiments part.

### Brief description of the figures

The accompanying figures are used to provide a further understanding of the present application and constitute a part of the specification. Together with the following specific embodiments, they are used to explain the present application but do not constitute a limitation to the present application. In the accompanying figures:
FIG. 1 is a schematic diagram of a preferred embodiment of the catalyst regeneration method and system of the present application;
FIG. 2 is a schematic diagram of another preferred embodiment of the catalyst regeneration method and system of the present application;
FIG. 3 is a schematic diagram of yet another preferred embodiment of the catalyst regeneration method and system of the present application; and
FIG. 4 is a schematic diagram of a further preferred embodiment of the catalyst regeneration method and system of the present application.

### Preferred Embodiments

The specific embodiments of the present application are described in details below in conjunction with the accompanying figures. It should be understood that the specific embodiments described here are only used to illustrate and explain the present application, and are not used to limit the present application.

As used herein, the expression "exemplary" means "serving as an example, embodiment, or illustration." Any embodiment described herein as "exemplary" is not necessarily to be construed as superior or better than other embodiments. Although various aspects of the embodiments are shown in the figures, the figures are not necessarily drawn to scale unless otherwise noted.

Any specific numerical value disclosed herein (including the endpoint of the numerical range) is not limited to the exact value of the numerical value, but should be understood to also cover values close to the exact value, such as all possible values within the range of ± 5% of the exact value. In addition, for the disclosed numerical range, one or more new numerical ranges may be obtained by any combination between the endpoint values of the range, between the endpoint values and the specific point values of the range, as well as between the specific point values, and these new numerical ranges should also be regarded as specifically disclosed herein.

In the present application, the so-called "upstream" and "downstream" are based on the flow direction of the reactants. For example, when the reactants flow from bottom to top, "upstream" means the position below, while "downstream" means the position above.

In the present application, it should be noted that the terms "upper", "lower", "inside", "outside", "front", "back", "left", "right", etc., indicate directions or positional relationships, which are based on the directions or positional relationships in the working state of the present application. They are only for the convenience of describing the present application and simplifying the description, and do not indicate or imply that the device or element referred to must have a specific direction, be constructed and operated in a specific direction. Therefore, they should not be understood as limitations on the present application.

In the present application, it should be noted that, unless otherwise clearly specified and limited, the terms "install", "connected", "connecting" and "communication" should be understood in a broad sense. For those of ordinary skills in the art, the specific meanings of the above terms in this application can be understood according to specific circumstances. For example, in this application, the term "connected" includes both the situation where two things are directly connected and the situation where two things are connected via one or more intermediate devices.

Unless otherwise specified, the terms used herein have the same meaning as commonly understood by those skilled in the art. If a term is defined herein and its definition is different from the commonly understood meaning in the art, the definition herein shall prevail.

In this application, except for the contents explicitly described, any matters or items not mentioned are directly applicable to those known in the art without any changes. Moreover, any embodiment described herein can be freely combined with one or more other embodiments described herein, and the technical solutions or technical ideas formed thereby are regarded as part of the original disclosure or original record of the present invention, and should not be regarded as new contents not disclosed or anticipated herein, unless those skilled in the art consider that the combination is obviously unreasonable.

All patents and non-patent literatures, including but not limited to textbooks and journal articles, mentioned herein are incorporated herein by reference in their entirety.

In addition, the technical features involved in different embodiments of the present application described below can be combined with each other as long as they do not conflict with each other.

As described above, in a first aspect, the present application provides a catalyst regeneration method suitable for a fluidized catalytic cracking unit comprising a catalytic cracking reactor and a catalyst regenerator, wherein the regeneration method comprises the following steps:
1) providing a gaseous biomass-derived fuel containing hydrogen and/or methane;
2) directly feeding the gaseous fuel into the catalyst regenerator without separation and purification;
3) introducing an oxygen-containing gas into the catalyst regenerator, wherein the oxygen content of the oxygen-containing gas is 14-28% by volume; and
4) feeding the catalyst to be regenerated from the catalytic cracking reactor into the catalyst regenerator, where it contacts with the gaseous fuel and the oxygen-containing gas for coke-burning and regeneration.

According to the present application, the gaseous biomass-derived fuel may be, for example, a gaseous fuel obtained by gasification or anaerobic fermentation of biomass.

The method provided in the present application uses gaseous biomass-derived fuel as a supplementary energy source, and burns in contact with oxygen-containing gas together with the catalyst to be generated to provide energy, which not only satisfies the heat balance of the catalytic cracking unit, but also reduces carbon emissions, and is also conducive to the separation and capture of carbon dioxide, becoming the source of negative carbon technology. The utilization of biomass is essentially an indirect solar energy utilization process. The carbon in the biomass comes from the carbon dioxide captured by plants from the atmosphere, rather than from fossil energy, and the energy consumed in the whole process also comes from solar energy. Therefore, the utilization of biomass energy is also a recycling of carbon elements, and is a carbon-neutral emission process. At the same time, the introduction of biomass into the power center of the catalytic cracking unit and supplying the operation of the unit with biomass energy consume renewable energy, and the carbon dioxide emitted does not come from fossil energy, which can fundamentally change the source of energy and achieve carbon emission reduction. Pure oxygen regeneration can also be used so that the regeneration flue gas contains only carbon dioxide and oxygen, which reduces the cost of separation and capture, and can achieve negative carbon emissions.

According to the present application, in a preferred embodiment, the biomass includes but is not limited to agricultural and forestry biomass, forestry biomass, aquatic plants, energy and cash crops, livestock and poultry manure, urban solid waste, domestic sewage and industrial organic sewage, etc. The agricultural and forestry biomass includes but is not limited to straw, husk, cotton stalks, etc., the forestry biomass includes but is not limited to firewood, fast-growing forests, forestry processing residues, etc., the aquatic plants include but are not limited to reeds, algae, etc., the energy and cash crops include cassava, rape, etc., the urban solid waste includes domestic garbage, commercial service garbage, etc., the domestic sewage and industrial organic sewage include cooling water, kitchen drainage, organic sewage discharged from brewing, food and other industries.

In a preferred embodiment, the operating temperature of the catalyst regenerator in step 4) is in the range of 550-750 °C, and the average catalyst residence time is 1.0-15.0 minutes.

In a preferred embodiment, the gaseous fuel in step 2) is injected into the catalyst regenerator through a gas distributor from a position not lower than the level of the catalyst to be regenerated inlet.

In certain preferred embodiments, the oxygen-containing gas is air or is oxygen diluted by recycled flue gas. Further preferably, when the oxygen-containing gas is air, the amount of the gaseous fuel introduced is no more than 13% by volume of the amount of air introduced, such as 3-13% by volume, or when the oxygen-containing gas is oxygen diluted by recycled flue gas, the amount of the gaseous fuel introduced is no more than 44% by volume, preferably 10-44 % by volume of the amount of oxygen introduced.

In certain preferred embodiments, the catalyst regenerator is a single-stage regenerator, and the operating conditions of the regenerator include: an operating temperature of 550-750 °C, an average catalyst residence time of 1.0-15.0 minutes, and a gas superficial linear velocity of 0.5-2.0 m/s.

Since the gaseous fuel produced from biomass has a high hydrogen content, a relatively large amount of steam will be generated when it burns in the catalyst regenerator. In order to avoid the adverse effects of the steam on the activity of the catalyst, the method of the present application is preferably carried out in a two-stage regenerator or a dual regenerator, and the gaseous fuel is only fed into the coke-burning section of the two-stage regenerator or the first regenerator of the dual regenerator, and the operating conditions in the two-stage regenerator and the dual regenerator are appropriately optimized.

In certain particularly preferred embodiments, the catalyst regenerator is a two-stage regenerator comprising a coke-burning section and a regeneration section which are in fluid communication, and in step 2), the gaseous fuel is fed into the coke-burning section and/or the regeneration section, preferably only into the coke-burning section, in step 3), the oxygen-containing gas is introduced into the bottom of the coke-burning section and the regeneration section respectively, and in step 4), the catalyst to be regenerated is fed into the coke-burning section. Further preferably, the operating conditions of the coke-burning section include: an operating temperature of 550-720 °C, an average catalyst residence time of 10.0-120.0 seconds, preferably 15.0-90.0 seconds, and a gas superficial linear velocity of 0.5-5.0 m/s, preferably 1.0-4.0 m/s; and the operating conditions of the regeneration section include: an operating temperature of 600-750 °C, an average catalyst residence time of 0.5-5.0 minutes, preferably 1.0-4.0 minutes, and a gas superficial linear velocity of 0.4-2.0 m/s, preferably 0.5-1.5 m/s. Further preferably, the operating temperature of the regeneration section is 10 - 150 °C higher than the operating temperature of the coke-burning section.

In certain further preferred embodiments, the operating conditions of the coke-burning section include: 600-700 °C, and an average catalyst residence time of 60.0-90.0 seconds; and the operating conditions of the regeneration section include: 650-720 °C, and an average catalyst residence time of 2.0-4.0 minutes, so that the steam generated by the combustion of the gaseous fuel can be used to age the catalyst during the catalyst regeneration process, thereby improving the selectivity of the catalyst for the target light olefin product, while avoiding excessive influence of steam on the catalyst activity, so as to be suitable for fluidized catalytic cracking process mainly for the production of chemicals such as light olefins and so on. In such preferred embodiments, the gaseous fuel can be injected only into the coke-burning section of the regenerator, or simultaneously into the coke-burning section and the regeneration section of the regenerator.

In some further preferred embodiments, the operating conditions of the coke-burning section include: 550-650 °C, and an average catalyst residence time of 15.0-60.0 seconds; and the operating conditions of the regeneration section include: 600-680 °C, and an average catalyst residence time is 1.0-3.0 minutes, thereby sufficiently weakening the adverse effect of steam on the catalyst activity, so that the obtained regenerated catalyst has a higher catalytic cracking activity, and is suitable for fluidized catalytic cracking mainly producing fuel oil. In such preferred embodiments, the gaseous fuel is only injected into the coke-burning section of the regenerator.

In other particularly preferred embodiments, the catalyst regenerator is a dual regenerator comprising a first regenerator and a second regenerator which are in fluid communication, and in step 2), the gaseous fuel is fed into the first regenerator and/or the second regenerator, preferably only into the first regenerator, in step 3), the oxygen-containing gas is introduced into the bottom of the first regenerator and the second regenerator, respectively, and in step 4), the catalyst to be regenerated is fed into the first regenerator. Further preferably, the operating conditions of the first regenerator include: an operating temperature of 550-720 °C, an average catalyst residence time of 20.0-240.0 seconds, preferably 30.0-150.0 seconds, and a gas superficial linear velocity of 0.5-5.0 m/s, preferably 1.0-4.0 m/s; and the operating conditions of the second regenerator include: an operating temperature of 600-750 °C, an average catalyst residence time of 0.5-5.0 minutes, preferably 1.0-4.0 minutes, and a gas superficial linear velocity of 0.4-2.0 m/s, preferably 0.5-1.5 m/s. Further preferably, the second regenerator is operated at a temperature 10-150 °C higher than the operating temperature of the first regenerator.

In certain further preferred embodiments, the operating conditions of the first regenerator include: 600-700 °C, and an average catalyst residence time of 90.0-180.0 seconds; and the operating conditions of the second regenerator include: 650-720 °C, and an average catalyst residence time of 2.0-4.0 minutes, so that the steam generated by the combustion of the gaseous fuel can be used to age the catalyst during the catalyst regeneration process, thereby improving the selectivity of the catalyst for the target light olefin product, while avoiding excessive influence of steam on the activity of the catalyst, so as to be suitable for the fluidized catalytic cracking process mainly producing chemicals such as light olefins, etc. In such preferred embodiments, the gaseous fuel can be injected only into the first regenerator, or simultaneously into the first regenerator and the second regenerator.

In other further preferred embodiments, the operating conditions of the first regenerator include: 550-650 °C, and an average catalyst residence time of 15.0-60.0 seconds; and the operating conditions of the second regenerator include: 600-680 °C, and an average catalyst residence time of 1.0-3.0 minutes, thereby sufficiently weakening the adverse effect of steam on the catalyst activity, so that the obtained regenerated catalyst has a higher catalytic cracking activity, so as to be suitable for fluidized catalytic cracking mainly producing fuel oil. In such preferred embodiments, the gaseous fuel can be injected only into the first regenerator.

In certain further preferred embodiments, the coke-burning ratio in the coke-burning section or the first regenerator is 40-70%, preferably 40-50%; and the coke-burning ratio in the regeneration section or the second regenerator is 30-60%, preferably 50-60%.

In certain preferred embodiments, the gaseous fuel is obtained by gasification of biomass and comprises, based on the total volume of the gaseous fuel, 12-60% of hydrogen, 15-30% of carbon monoxide and 3-8% of methane, and the remainder is carbon dioxide and/or nitrogen.

In a further preferred embodiment, the step 1) further comprises: gasifying the biomass in the presence of a gasification medium at a gasification temperature of 500-1500 °C, wherein the gasification medium is selected from air, oxygen/oxygen-enriched gas, and steam.

In other preferred embodiments, the gaseous fuel is obtained through anaerobic fermentation of biomass and comprises 40-100 % by volume of methane based on the total volume of the gaseous fuel.

In a further preferred embodiment, the step 1) further comprises: subjecting the biomass to anaerobic fermentation in a closed fermentation tank, and the fermentation temperature is not higher than 60 °C.

In some further preferred embodiments, the biomass is pretreated before gasification or anaerobic fermentation. The biomass pretreatment process is well known to those skilled in the art. Preferably, the pretreatment is selected from one or more of grinding, drying, extrusion, steam explosion, acid treatment, alkali treatment and microbial pretreatment.

In certain preferred embodiments, the temperature in the catalyst regenerator is controlled not to exceed 750 °C by a heat extraction system including one or more internal heat extractors and/or external heat extractors. Further preferably, the heat extraction system uses the heat extracted from the catalyst regenerator to generate high-pressure steam, which is then exported to other devices for energy supply. In such preferred embodiments, the present application can use the energy generated by the regeneration system of the catalytic cracking unit to supply other operating units, becoming the power center of the refinery, and fundamentally reducing the carbon emissions of the refinery. The present application introduces biomass into the power center of the catalytic cracking unit, and uses biomass energy to supply the operation of the unit. The carbon dioxide emitted does not come from fossil energy, which can fundamentally change the source of energy and achieve carbon emission reduction.

In a second aspect, the present application provides a catalyst regeneration system suitable for a fluidized catalytic cracking unit, comprising a biomass processing unit and a catalyst regeneration unit, wherein:
the biomass processing unit is used to process biomass, for example by gasification or anaerobic fermentation, to obtain a gaseous fuel containing hydrogen and/or methane, and comprises a gaseous fuel generator and a gaseous fuel storage tank, wherein the gaseous fuel generator is preferably selected from a biomass gasifier, a biomass anaerobic fermentation tank or a combination thereof, and has a biomass inlet and a gaseous product outlet, wherein the gaseous fuel storage tank has an inlet and a gaseous fuel outlet, wherein the gaseous product outlet of the gaseous fuel generator is connected to the inlet of the gaseous fuel storage tank;
wherein the catalyst regeneration unit is used to regenerate the catalyst to be regenerated from the catalytic cracking reactor, and comprises a catalyst regenerator, wherein the catalyst regenerator has a catalyst to be regenerated inlet, an oxygen-containing gas inlet, a gaseous fuel inlet, a regeneration flue gas outlet, and a regenerated catalyst outlet, and
wherein the gaseous fuel outlet of the gaseous fuel storage tank is connected to the gaseous fuel inlet of the catalyst regenerator through a pipeline.

In a preferred embodiment, the biomass processing unit further comprises a biomass preprocessor and an optional gaseous product dryer, wherein the biomass preprocessor is used to pretreat the biomass, wherein the pretreatment is selected from one or more of grinding, drying, extrusion, steam explosion, acid treatment, alkali treatment and microbial pretreatment, and the gaseous product dryer is used to dry the gaseous product obtained from the biomass anaerobic fermentation tank.

In certain preferred embodiments, the catalyst regenerator comprises a coke-burning section and a dense phase regeneration section, the dense phase regeneration section being located above the coke-burning section, and wherein the outlet of the coke-burning section is accommodated inside the dense phase regeneration section, so that the coke-burning section is in fluid communication with the dense phase regeneration section;
wherein the coke-burning section is provided with:
a first oxygen-containing gas inlet, which is provided at the bottom of the coke-burning section and is used to feed an oxygen-containing gas into the coke-burning section;
the gaseous fuel inlet, which is provided above the first oxygen-containing gas inlet and is used to feed the gaseous fuel;
a gas distributor configured to distribute the gaseous fuel fed through the gaseous fuel inlet;
the catalyst to be regenerated inlet, which is used to transport the catalyst to be regenerated from the catalytic cracking reactor to the interior of the coke-burning section; and
an optional first recycled flue gas inlet, which is used to recycle a portion of the flue gas discharged from the dense phase regeneration section back into the interior of the coke-burning section;
wherein the dense phase regeneration section is provided with:
   a second oxygen-containing gas inlet, which is provided at the bottom of the dense phase regeneration section and is used to feed an oxygen-containing gas into the dense phase regeneration section;
   an optional second gaseous fuel inlet, which is provided above the second oxygen-containing gas inlet and is used to feed the gaseous fuel into the dense phase regeneration section;
   an optional second gas distributor configured to distribute the gaseous fuel fed through the second gaseous fuel inlet;
   the regeneration flue gas outlet, which is provided at the top of the dense phase regeneration section and is used to discharge the regeneration flue gas in the dense phase regeneration section;
   the regenerated catalyst outlet, which is used to return the regenerated catalyst to the catalytic cracking reactor; and
   an optional second recycled flue gas inlet, which is used to recycle a portion of the flue gas discharged from the dense phase regeneration section back into the dense phase regeneration section;
   optionally, the dense phase regeneration section is further provided with a heat extractor for transferring heat to the outside of the regenerator.
   In some other preferred embodiments, the catalyst regenerator comprises a first regenerator and a second regenerator, wherein the second regenerator is located downstream of the first regenerator, wherein the first regenerator and the second regenerator are connected by a catalyst transport pipe to deliver the catalyst material partially regenerated by the first regenerator to the second regenerator;
   wherein the first regenerator is provided with:
      a first oxygen-containing gas inlet, which is provided at the bottom of the first regenerator and is used to feed an oxygen-containing gas into the first regenerator;
      the gaseous fuel inlet, which is provided above the first oxygen-containing gas inlet and is used to feed the gaseous fuel;
      a gas distributor configured to distribute the gaseous fuel fed through the gaseous fuel inlet;
      the catalyst to be regenerated inlet, which is used to transport the catalyst to be regenerated from the catalytic cracking reactor to the interior of the first regenerator;
      a first regeneration flue gas outlet, which is provided at the top of the first regenerator and is used to discharge the regeneration flue gas in the first regenerator; and
      an optional first recycled flue gas inlet, which is provided at the bottom of the first regenerator and in communication with the first regeneration flue gas outlet, for recycling a portion of the flue gas discharged from the first regenerator back to the first regenerator,
      wherein the second regenerator is provided with:
         a second oxygen-containing gas inlet, which is provided at the bottom of the second regenerator and is used to feed an oxygen-containing gas into the second regenerator;
         an optional second gaseous fuel inlet, which is provided above the second oxygen-containing gas inlet and is used to feed the gaseous fuel into the second regenerator;
         an optional second gas distributor configured to distribute the gaseous fuel fed through the second gaseous fuel inlet;
         the regenerated catalyst outlet, which is used to return the regenerated catalyst to the catalytic cracking reactor;
         a second regeneration flue gas outlet, which is provided at the top of the second regenerator and is used to discharge the regeneration flue gas in the second regenerator; and
         an optional second recycled flue gas inlet, which is provided at the bottom of the second regenerator and is connected to the first regeneration flue gas outlet and/or the second regeneration flue gas outlet, for recycling a portion of the flue gas back to the second regenerator.

The preferred embodiments of the regeneration method and system of the present application will be further described in details below in conjunction with the accompanying figures.

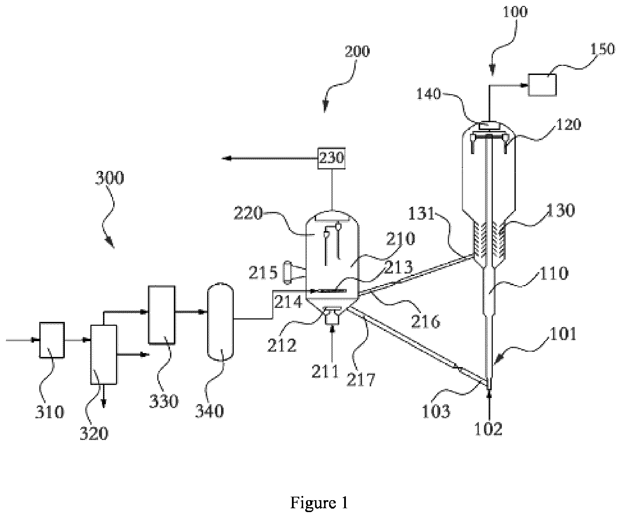

As shown in FIG. 1, FIG. 2, FIG. 3 and FIG. 4, the catalyst regeneration system of the present application is suitable for regenerating the catalyst to be regenerated from the catalytic cracking reaction unit 100, and includes: biomass processing units 300 and 600 and catalyst regeneration units 200, 400, 500, 700.

As shown in Fig. 1 to FIG. 4, in the catalytic cracking reaction system 100, the catalytic cracking reactor 110 is used to carry out a catalytic cracking reaction: the bottom inlet 102 thereof is fed with a lifting medium to lift the regenerated catalyst (from the regenerator) entering through the regenerated catalyst inlet 103; the feedstock oil entering from the feedstock oil inlet 101 contacts the catalyst to carry out a catalytic cracking reaction. The oil and gas products of the reaction are separated by the oil catalyst separation device 120, and the separated oil and gas products are gathered by the gas collecting chamber 140 and then transported into the product separation device 150 for separation to obtain various products. The separated catalyst to be regenerated is stripped by the stripping section 130 of the settler and then transported to the catalyst regeneration unit through the catalyst to be regenerated outlet 131, thereby realizing recycling. The catalytic cracking reactor 110 applicable to the present application can be various reactors commonly used in the art, such as a riser reactor, a fluidized bed reactor, a variable diameter reactor and a combination thereof.

As shown in figures 1-2, the biomass processing unit 300 includes:
a biomass preprocessor 310, which is used to pretreat the biomass,
a biomass anaerobic fermentation tank 320, which is used to perform anaerobic fermentation on the pretreated biomass to obtain a gaseous fermentation product,
a gaseous product dryer 330, which is used to dry the gaseous fermentation product obtained from the biomass anaerobic fermentation tank, and
a gaseous fuel storage tank 340, which is used to store the gaseous product.

As shown in the figure, the anaerobic fermentation process of biomass can be carried out in the biomass anaerobic fermentation tank 320, such as a closed fermentation tank, wherein the fermentation substrate can be a mixed biomass raw material, and the fermentation temperature is not higher than 60 °C. According to the present application, in a more preferred embodiment, urea, biochar, etc. can be added to enhance the fermentation process.

According to the present application, the anaerobic fermentation products mainly include methane, carbon dioxide, water, etc. Since water has an adverse effect on the subsequent process, drying process is needed. Drying can be performed in the gaseous product dryer 330 to remove moisture from the fermentation product, so that the water content is less than 1.0 g/m³.

After drying, the dried gaseous product is stored in the gaseous fuel storage tank 340 for subsequent processes. In the dried gaseous product, methane can account for more than 40%, such as 40-50%, can account for more than 60%, and can account for more than 75%, based on the total volume of the gaseous product.

According to the present application, the energy consumed in the anaerobic fermentation process comes from other products of the fermentation process or at least partially/entirely from renewable energy sources such as solar energy, green electricity, nuclear energy, etc., thereby reducing carbon emissions throughout the life cycle.

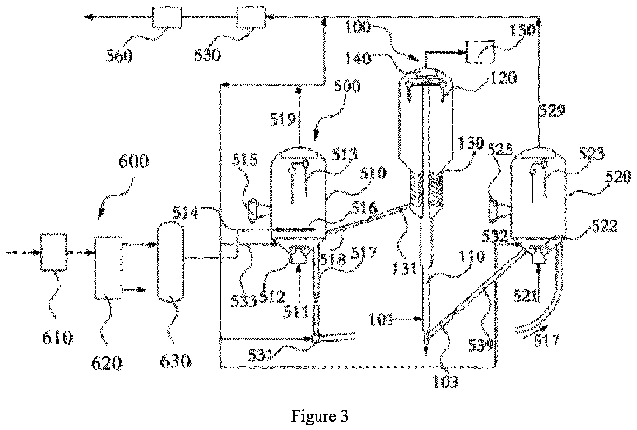

As shown in figures 3-4, the biomass processing unit 600 includes:
a biomass preprocessor 610, which is used to pretreat the biomass,
a biomass gasifier 620, which is used to gasify the pretreated biomass to obtain a gaseous product, and
a gaseous fuel storage tank 630, which is used to store the gaseous product.

As shown in the figures, the biomass can be transported to the biomass preprocessor 610, and after being processed by grinding, drying, etc., it is transported to the biomass gasifier 620. In the biomass gasifier 620, the biomass is gasified to obtain a gaseous product, which is transported to the gaseous fuel storage tank 630 for subsequent regeneration processing, and the remaining products are drawn out or sent to other devices for processing. In a specific embodiment, the biomass material is ground or crushed into a granular material with a particle size of 0.2-40mm, and then dried, so that its water content is less than 10%.

Afterwards, the biomass is gasified. The gasification temperature can be in the range of 500-1500 °C. The gasification medium used in the gasification process can be selected from air, oxygen/oxygen-enriched gas, steam, etc. According to the present application, the energy consumed in the biomass gasification process can come from other products of the gasification process, or at least partly/entirely from renewable energy sources such as solar energy, green electricity, nuclear energy, etc., to reduce carbon emissions throughout the life cycle.

According to the present application, the main components of the obtained gaseous product are hydrogen, carbon monoxide, carbon dioxide and a small amount of methane gas. Generally speaking, hydrogen accounts for 12-60%, carbon monoxide accounts for 15-30%, and methane accounts for 3-8%, based on the total volume of the gaseous product. The remaining components can be carbon dioxide and/or nitrogen.

FIG. 1 shows a schematic diagram of a first preferred embodiment of the catalyst regeneration method and system of the present application, wherein the regeneration process is a single-stage regeneration. As shown in FIG. 1, the regeneration unit 200 includes a regenerator 210, wherein the regenerator 210 is provided with:
an oxygen-containing gas inlet 211 for feeding an oxygen-containing gas into the regenerator, wherein the oxygen-containing gas inlet 211 is provided at the bottom of the regenerator;
a distribution plate 212 configured to distribute the oxygen-containing gas fed through the oxygen-containing gas inlet 211;
a gaseous fuel inlet 214, which is provided above the distribution plate 212 and is used to feed a gaseous fuel (the gaseous product from the gaseous fuel storage tank 340);
a gas distributor 213 configured to distribute the gaseous fuel fed through the gaseous fuel inlet 214;
a catalyst to be regenerated inlet 216, which is used to transport the catalyst to be regenerated from the catalytic cracking reactor to the interior of the regenerator;
a regenerated catalyst outlet 217, which is used to transport the regenerated catalyst to the catalytic cracking reactor.

The gaseous fuel storage tank 340 is connected to the gaseous fuel inlet 214, so that the gaseous fuel is transported to the interior of the regenerator, burns and regenerates the catalyst to be regenerated.

The distribution plate 212 and the gas distributor 213 are both provided inside the regenerator 210 to evenly distribute the oxygen-containing gas and the gaseous fuel in the catalyst to be regenerated inside the regenerator, so that the catalyst can burn evenly during the regeneration process to avoid local overheating. Generally, the distribution plate 212 and the gas distributor 213 are both provided at the lower part of inside of the regenerator 210 to make the catalyst to be regenerated in a fluidized state inside the regenerator during the regeneration process in the regenerator.

The regenerator is a dense bed, and the catalyst bed density is 300-700 kg/m³. The gaseous fuel is injected through the gas distributor at a level not lower than the level of the catalyst to be regenerated inlet. In this embodiment, the gas distributor 213 is provided at the lower part of the dense bed section of the regenerator to better distribute the gaseous fuel evenly.

In a specific embodiment, the conditions of the regeneration process are: the oxygen-containing gas is air, the regeneration temperature is 550-750 °C, the average catalyst residence time is 1.0-15.0 minutes, and the gas superficial linear velocity is 0.7-2.0 m/s.

The gaseous fuel and the catalyst to be regenerated are transported together to the bottom of the regenerator to contact with the oxygen-containing gas for coke-burning regeneration and energy supply. Due to the injection of the gaseous fuel, a large amount of heat will be generated during the regeneration process. If the temperature in the regenerator is too high, it will adversely affect the activity of the catalyst. Therefore, the regeneration unit 200 is also equipped with a heat extractor 215 for removing excess heat from the regenerator. The heat extractor can be an internal heat extractor (arranged inside the regenerator) or/and an external heat extractor (arranged outside the regenerator). The heat extractor is one or more, which can be used to supply the excess energy generated by the regenerator to other devices. The excess heat of the regeneration system can be used to generate high-pressure steam through the heat extractor, which is then exported to other devices for energy supply. In a specific embodiment, by providing a heat extractor, the regenerator bed temperature is controlled not to exceed 750 °C, for example, not to exceed 720 °C.

As shown in FIG. 1, the regeneration unit 200 further includes a cyclone separator 220, and the regeneration flue gas leaves the regenerator through the cyclone separator 220 and enters the flue gas energy recovery system 230 to recover energy. The cyclone separator 220 may be provided inside the regenerator 210.

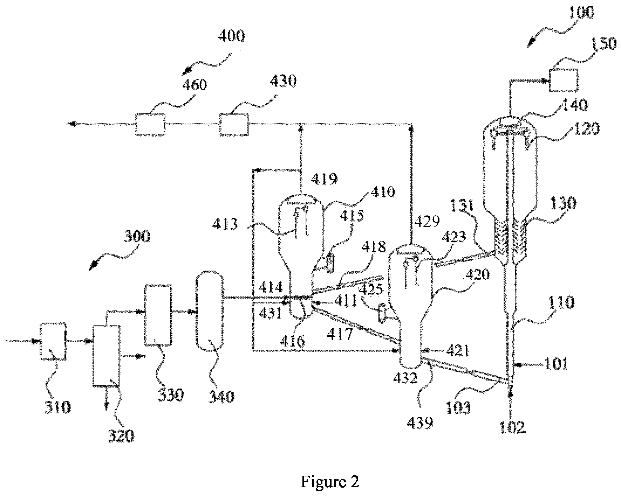

FIG. 2 shows a schematic diagram of a second preferred embodiment of the catalyst regeneration method and system of the present application, wherein the regeneration process adopts dual regenerator regeneration. The regeneration unit 400 includes a first regenerator 410 and a second regenerator 420. The two regenerators are connected in series, and the second regenerator 420 is located downstream of the first regenerator 410 and connected by a catalyst transport pipe 417, so that the catalyst material of the first regenerator is transported to the second regenerator.

The first regenerator 410 is provided with:
a first oxygen-containing gas inlet 411, which is provided at the bottom of the first regenerator and is used to feed oxygen into the first regenerator;
a gaseous fuel inlet 414, which is provided above the first oxygen-containing gas inlet and is used to feed the gaseous fuel;
a gas distributor 416 configured to distribute the gaseous fuel fed through the gaseous fuel inlet; and
a catalyst to be regenerated inlet 418 (connected to a to-be-regenerated inclined pipe), which is used to transport the catalyst to be regenerated from the catalytic cracking reactor to the interior of the first regenerator;
a first flue gas outlet 419, which is provided at the top of the first regenerator;
wherein the second regenerator 420 is provided with:
   a second oxygen-containing gas inlet 421, which is provided at the bottom of the second regenerator and is used to feed oxygen into the second regenerator;
   a regenerated catalyst outlet 439 (connected to a regeneration inclined pipe), which is used to transport the regenerated catalyst to the catalytic cracking reactor; and
   a second flue gas outlet 429, which is provided at the top of the second regenerator.

During the regeneration operation, the gaseous fuel is introduced into the first regenerator 410 from the gaseous fuel inlet 414 through the gas distributor 416, and the catalyst to be regenerated from the catalytic cracking reactor enters the first regenerator through the to-be-regenerated inclined pipe (connected to a catalyst to be regenerated outlet 131) and through the catalyst to be regenerated inlet 418, and contacts with the oxygen entering from the first oxygen-containing gas inlet 411, and performs a partial coking-burning reaction (first stage regeneration) in the first regenerator; the partially regenerated catalyst is transported to the second regenerator 420 through the catalyst transport pipe 417, and after contacting with the oxygen entering from the second oxygen-containing gas inlet 421, a coke-burning reaction occurs to complete regeneration.

In a specific embodiment, a first recycled flue gas inlet 431 is provided at the bottom of the first regenerator, and the first recycled flue gas inlet 431 is connected to the first flue gas outlet 419, so that a portion of the flue gas discharged from the first regenerator enters the first regenerator through the first recycled flue gas inlet.

In a specific embodiment, a second recycled flue gas inlet 432 is provided at the bottom of the second regenerator, and the second recycled flue gas inlet 432 is connected to the first flue gas outlet 419, so that a portion of the flue gas discharged from the first regenerator enters the second regenerator through the second recycled flue gas inlet.

In a specific embodiment, the bottom of the first regenerator and the bottom of the second regenerator are connected via the catalyst transport pipe 417.

Thus, after the flue gas generated by the first regenerator is separated by a cyclone separator 413, a portion of it enters the flue gas energy recovery device 430 to recover energy; the other portion of the flue gas is divided into two parts and circulated back to the regeneration unit, one part is transported to the first regenerator to dilute the introduced oxygen; the other part is transported to the second regenerator to dilute the oxygen.

The flue gas from the second regenerator is separated by a cyclone separator 423, enters the flue gas energy recovery device 430 through the second recycled flue gas outlet 429 to recover energy, and is transported to the carbon dioxide separation system 460 to capture the carbon dioxide gas therein. The regenerated catalyst is circulated back to the catalytic cracking reactor through the regenerated catalyst inlet 439 and the regeneration inclined pipe (connected to the regenerated catalyst inlet 103).

In a specific embodiment, the operating conditions of the first regenerator are: a temperature of 550 °C -700 °C, an average catalyst residence time of 20.0-240.0 seconds, and a gas superficial linear velocity of 0.5-5.0 m/s. The operating conditions of the second regenerator are: a temperature of 600 °C -750 °C, an average catalyst residence time of 0.5-5.0 minutes, and a gas superficial linear velocity of 0.4-2.0 m/s.

Due to the injection of gaseous fuel, a large amount of heat will be generated during the regeneration process. If the temperature in the regenerator is too high, it will adversely affect the activity of the catalyst. Therefore, the regeneration unit 400 is also equipped with heat extractors 415, 425 for transferring heat to the outside of the first regenerator and the second regenerator. The heat extractor can be an internal heat extractor (arranged inside the regenerator) or/and an external heat extractor (arranged outside the regenerator), and the heat extractor is one or more, extracting the excess energy from the first regenerator and the second regenerator to supply other devices. The excess heat of the regeneration system can be used to generate high-pressure steam through the heat extractor, and then exported to other devices for energy supply. In a specific embodiment, by providing the heat extractor 415, the bed temperature of the first regenerator is controlled not to exceed 700 °C, and by providing the heat extractor 425, the bed temperature of the second regenerator is controlled not to exceed 750 °C, for example, not to exceed 720 °C.

The gaseous product obtained from biomass contains a large amount of methane, which can account for more than 40%, such as 40-50%, can account for more than 60%, and can account for more than 75%, based on the total volume of the gaseous product. The molecular formula of methane is CH₄, and the hydrogen content thereof is as high as 25%. It is generally believed in the art that excessive steam in the regeneration process is detrimental to the activity of the catalytic cracking catalyst, and a mixed gas with a high hydrogen content is generally not used as a supplementary fuel for the regeneration process. In the preferred embodiment shown in FIG. 2, the regeneration process adopts a pure oxygen regeneration process and adopts a dual regenerator regeneration, which can better protect the catalyst and avoid the influence of steam. In some further preferred embodiments, by further optimizing the operating conditions of the first and second regenerators, the steam can be used to age the catalyst to improve product selectivity.

In some specific embodiments, the regeneration method performed in the regeneration system including the regeneration unit 400 includes:
1) transporting the biomass after pretreatment to a biomass anaerobic fermentation system for anaerobic fermentation to obtain a gaseous fermentation product;
2) drying the gaseous fermentation product and storing the dried gaseous fuel in a gaseous fuel storage tank;
3) transporting the gaseous fuel to the interior of the first regenerator to contact with the catalyst to be regenerated from the catalytic cracking reactor and oxygen, so that the catalyst to be regenerated undergoes partial coke-burning;
4) feeding the material from the first regenerator to the second regenerator, and injecting oxygen into the second regenerator through the second oxygen-containing gas inlet to completely regenerate the catalyst.

FIG. 3 shows a schematic diagram of a third preferred embodiment of the catalyst regeneration method and system of the present application, wherein the regeneration process adopts a dual regenerator regeneration. The regeneration unit 500 includes a first regenerator 510 and a second regenerator 520 that are connected in series, and the second regenerator 520 is located downstream of the first regenerator 510, connected by a U-shaped catalyst transport pipe 517, so that the catalyst material of the first regenerator is transported to the second regenerator.

The first regenerator 510 is provided with:
a first oxygen-containing gas inlet 511, which is provided at the bottom of the first regenerator and is used to feed oxygen into the first regenerator;
a gaseous fuel inlet 514, which is provided above the first oxygen-containing gas inlet and is used to feed the gaseous fuel;
a gas distributor 516 configured to distribute the gaseous fuel fed through the gaseous fuel inlet; and
a catalyst to be regenerated inlet 518 (connected to a to-be-regenerated inclined pipe), which is used to transport the catalyst to be regenerated from the catalytic cracking reactor to the interior of the first regenerator;
a first flue gas outlet 519, which is provided at the top of the first regenerator;
wherein the second regenerator 520 is provided with:
   a second oxygen-containing gas inlet 521, which is provided at the bottom of the second regenerator and is used to feed oxygen into the second regenerator;
   a regenerated catalyst outlet 539 (connected to a regeneration inclined pipe), which is used to transport the regenerated catalyst to the catalytic cracking reactor; and
   a second flue gas outlet 529, which is provided at the top of the second regenerator.

During the regeneration operation, the gaseous fuel is introduced into the first regenerator 510 from the gaseous fuel inlet 514 through the gas distributor 516, and the catalyst to be regenerated from the catalytic cracking reactor enters the first regenerator through the to-be-regenerated inclined pipe (connected to a catalyst to be regenerated outlet 131) and through the catalyst to be regenerated inlet 518, and contacts with the oxygen entering from the oxygen-containing gas inlet 511 through the fluid distribution plate 512, and performs a partial coking-burning reaction (first stage regeneration) in the first regenerator; the partially regenerated catalyst is transported to the second regenerator 520 through the U-shaped catalyst transport pipe 517, and after contacting with the oxygen entering from the oxygen-containing gas inlet 521 through the fluid distribution plate 522, a coke-burning reaction occurs for complete regeneration.

In a specific embodiment, a first recycled flue gas inlet 533 is provided at the bottom of the first regenerator, and the first recycled flue gas inlet 533 is connected to the first flue gas outlet 519, so that a portion of the flue gas discharged from the first regenerator enters the first regenerator through the first recycled flue gas inlet.

In a specific embodiment, a second recycled flue gas inlet 532 is provided at the bottom of the second regenerator, and the second recycled flue gas inlet 532 is connected to the first flue gas outlet 519, so that a portion of the flue gas discharged from the first regenerator enters the second regenerator through the second recycled flue gas inlet.

In a specific embodiment, the bottom of the first regenerator and the bottom of the second regenerator are connected via the U-shaped catalyst transport pipe 517.

The U-shaped catalyst transport pipe is provided with an opening 531, which is in communication with the first flue gas outlet 519, so that a portion of the flue gas discharged from the first regenerator enters the U-shaped catalyst transport pipe through the opening.

Thus, after the flue gas generated by the first regenerator is separated by a cyclone separator 513, a portion of it enters the flue gas energy recovery device 530 to recover energy; the other portion of the flue gas is divided into three parts and circulated back to the regeneration unit. One part is transported to the first regenerator to dilute the introduced oxygen; one part is transported to the U-shaped catalyst transport pipe for transporting the semi-regenerated catalyst to the second regenerator; and the last part is transported to the second regenerator to dilute the introduced oxygen. The amount of oxygen and/or recycled flue gas is controlled so that the oxygen concentration in the mixed gas in the first regenerator and the second regenerator is not higher than 28%. Coke-burning under this atmosphere improves the coke-burning intensity; the intake gas does not contain nitrogen, which can reduce the energy consumed by gas preheating; and the flue gas at the outlet of the regenerator has a higher carbon dioxide concentration, which is conducive to the separation and capture of carbon dioxide.

The flue gas from the second regenerator is separated by a cyclone separator 523, then enters the flue gas energy recovery device 530 through the second recycled flue gas outlet 529 to recover energy, and is transported to the carbon dioxide separation system 560 to capture the carbon dioxide gas therein. The regenerated catalyst is circulated back to the catalytic cracking reactor through the regenerated catalyst inlet 539 and the regeneration inclined pipe (connected to the regenerated catalyst inlet 103).

In a specific embodiment, the operating conditions of the first regenerator are: a temperature of 550 °C -700 °C, an average catalyst residence time of 20.0-240.0 seconds, and a gas superficial linear velocity of 0.5-5.0 m/s. The operating conditions of the second regenerator are: a temperature of 600 °C -750 °C, an average catalyst residence time of 0.5-5.0 minutes, and a gas superficial linear velocity of 0.4-2.0 m/s.

Due to the injection of gaseous fuel, a large amount of heat will be generated during the regeneration process. If the temperature in the regenerator is too high, it will adversely affect the activity of the catalyst. Therefore, the regeneration unit 500 is also equipped with heat extractors 515, 525, which are used to remove excess heat from the first regenerator and the second regenerator, and optionally transport heat to the outside. The heat extractor can be an internal heat extractor (arranged inside the regenerator) or/and an external heat extractor (arranged outside the regenerator), and the heat extractor is one or more, extracting the excess energy generated by the first regenerator and the second regenerator to supply other devices. The excess heat of the regeneration system can be used to generate high-pressure steam through the heat extractors, and then exported to other devices for energy supply. In a specific embodiment, by providing the heat extractor 515, the bed temperature of the first regenerator is controlled not to exceed 700 °C; by providing the heat extractor 525, the bed temperature of the second regenerator is controlled not to exceed 750 °C, for example, not to exceed 720 °C.

The gaseous product obtained by biomass gasification contains a certain amount of hydrogen, for example, hydrogen accounts for 12-60%, carbon monoxide accounts for 15-30%, and methane accounts for 3-8%, based on the total volume of the gaseous product. It is generally believed in the art that excessive steam in the regeneration process is detrimental to the activity of the catalytic cracking catalyst, and thus a mixed gas containing hydrogen is generally not used as a supplementary fuel for the regeneration process. In the preferred embodiment shown in FIG. 3, the regeneration process adopts a pure oxygen regeneration process and adopts a dual regenerator regeneration, which can better protect the catalyst and avoid the influence of steam. In certain further preferred embodiments, by further optimizing the operating conditions of the first and second regenerators, the steam can be used to age the catalyst to improve product selectivity.

In certain specific embodiments, the regeneration method performed in the regeneration system including the regeneration unit 500 includes:
1) transporting the biomass after pretreatment to a gasification device for gasification treatment to obtain a gaseous product;
2) transporting the gaseous product to the interior of the first regenerator to contact with the catalyst to be regenerated from the catalytic cracking reactor and oxygen, so that the catalyst to be regenerated undergoes partial coke-burning;
3) feeding the material from the first regenerator to the second regenerator, and injecting oxygen into the second regenerator to completely regenerate the catalyst.

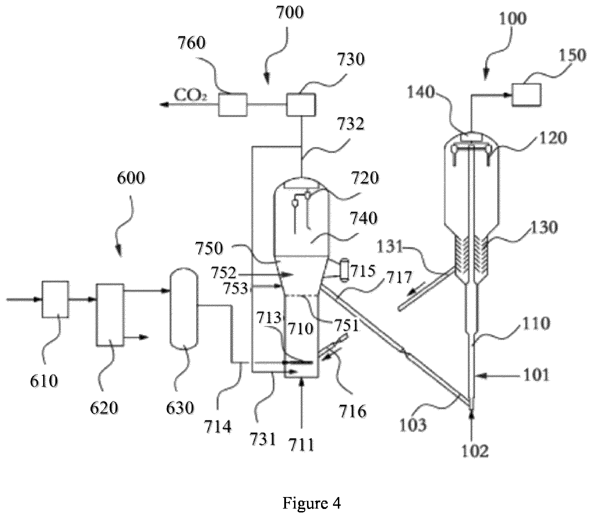

FIG. 4 shows a schematic diagram of a fourth preferred embodiment of the catalyst regeneration method and system of the present application, wherein the regeneration process adopts two-stage regeneration. As shown in the figure, the regeneration unit 700 includes a regenerator 740, and the regenerator 740 includes a coke-burning section 710 and a regeneration section 750, wherein the regeneration section 750 is in fluid communication with the coke-burning section 710 and is located above the coke-burning section 710; the regeneration section 750 is separated from the coke-burning section 710 by a fluid distribution plate 751. Thus, the regeneration section 750 and the coke-burning section 710 are connected in series. After the material from the coke-burning section 710 is distributed by the fluid distribution plate 751, it enters the regeneration section 750 for complete regeneration.

The coke-burning section 710 is provided with:
a first oxygen-containing gas inlet 711 for feeding oxygen into the coke-burning section, wherein the first oxygen-containing gas inlet is provided at the bottom of the coke-burning section;
a gaseous fuel inlet 714, which is provided above the first oxygen-containing gas inlet and is used to feed the gaseous fuel;
a gas distributor 713 configured to distribute the gaseous fuel fed through the gaseous fuel inlet;
a catalyst to be regenerated inlet 716, which is used to transport the catalyst to be regenerated from the catalytic cracking reactor to the interior of the coke-burning section; and
a first recycled flue gas inlet 731, which is used to recycle a portion of the flue gas recovered from the regeneration section back into the interior of the coke-burning section.

Thus, the catalyst to be regenerated is initially coke-burned in the coke-burning section 710 and the gaseous fuel is partially burned, thereby avoiding the hydrothermal deactivation of the catalyst by water generated during the hydrogen combustion process.

The regeneration section 750 is provided with:
a second oxygen-containing gas inlet 752, which is provided at the bottom of the regeneration section and is used to feed oxygen into the regeneration section;
a second recycled flue gas inlet 753, which is used to recycle a portion of the flue gas recovered from the regeneration section back into the interior of the regeneration section; and
a flue gas outlet 732, which is provided at the top of the regeneration section;
wherein the regeneration section 750 is also provided with a heat extractor 715 for transferring heat to the outside of the regenerator.

During the regeneration operation, the gaseous fuel from the gaseous fuel storage tank 630 is fed to the coke-burning section 710 through the gaseous fuel inlet 714 and the gas distributor 713. The catalyst to be regenerated from the catalytic cracking reactor enters the coke-burning section 710 through the catalyst to be regenerated inlet 716, contacts the oxygen entering through the first oxygen-containing gas inlet 711, and undergoes a partial coke-burning reaction in the coke-burning section; then, after passing through the fluid distribution plate 751, it enters the regeneration section 750 for complete regeneration. At this time, pure oxygen is fed through the second oxygen-containing gas inlet 752 to contact the partially coke-burned catalyst, so that the catalyst and the incompletely regeneration flue gas are further regenerated and burned. After the regenerated catalyst is separated by a cyclone separator 720, it falls back to the regeneration section, is discharged through the catalyst outlet 717, and is circulated back to the catalytic cracking reactor. A part of the flue gas discharged through the flue gas outlet 732 is recovered by the flue gas energy recovery system 730, and then separated by the carbon dioxide separation system 760 to achieve carbon dioxide capture; the other part of the flue gas is circulated back to the bottom of the coke-burning section.

In a specific embodiment, the operating conditions of the coke-burning section are: a temperature of 550-720 °C, an average catalyst residence time of 10.0-120.0 seconds, preferably 15.0-90.0 seconds, a gas superficial linear velocity of 0.5-5.0 m/s, preferably 1.0-4.0 m/s.

In a specific embodiment, the operating conditions of the regeneration section are: a temperature of 600-750 °C, an average catalyst residence time of 0.5-5.0 minutes, preferably 1.0-4.0 minutes, a gas superficial linear velocity of 0.4-2.0 m/s, preferably 0.5-1.5 m/s. The catalyst is completely regenerated in the regeneration section, and the gaseous fuel derived from biomass is completely burned in the regeneration section. In a specific embodiment, the regeneration section is a dense bed, and its catalyst density is 300-700 kg/m³.

In a specific embodiment, the gases fed through the first oxygen-containing gas inlet 711 and the second oxygen-containing gas inlet 752 are both oxygen. However, the oxygen fed through the first oxygen-containing gas inlet 711 will be mixed with the recycled flue gas after entering the coke-burning section to form an oxygen-carbon dioxide mixed gas. The amount of oxygen and/or recycled flue gas is controlled, so that the oxygen concentration in the mixed gas does not exceed 28% by volume. Similarly, the oxygen fed through the second oxygen-containing gas inlet 752 will be mixed with the recycled flue gas and others after entering the regeneration section to form an oxygen-carbon dioxide mixed gas. The amount of oxygen and/or recycled flue gas will be controlled, so that the oxygen concentration in the mixed gas is not higher than 28%. Coke-burning in this atmosphere improves the coke-burning intensity; the intake gas does not contain nitrogen, which can reduce the energy consumed by gas preheating; and the flue gas at the outlet of the regenerator has a higher carbon dioxide concentration, which is convenient for the separation and capture of carbon dioxide.

In a specific embodiment, the coke-burning ratio in the coke-burning section is 40-70%; the coke-burning ratio in the regeneration section is 30-60%. In a specific embodiment, pure oxygen is used for regeneration, and the regeneration flue gas contains only carbon dioxide and oxygen, which is convenient for separating and capturing carbon dioxide for further conversion and utilization, thereby achieving negative carbon emissions. According to the present application, the gaseous fuel obtained by biomass gasification is introduced from a gas distributor.

In the preferred embodiment shown in FIG. 4, the regeneration process adopts a pure oxygen regeneration process, and a two-stage regeneration is performed in a coke-burning section and a regeneration section that are connected in series, which can improve the coke-burning intensity and achieve a better regeneration effect, while also weaken the influence of steam on the catalyst. In certain further preferred embodiments, by further optimizing the operating conditions of the coke-burning section and the regeneration section, the steam can be used to age the catalyst to improve product selectivity.

Due to the injection of gaseous fuel, a large amount of heat will be generated during the regeneration process. If the temperature in the regenerator is too high, it will adversely affect the activity of the catalyst. Therefore, the regeneration unit 700 is also equipped with a heat extractor 715 for transferring heat to the outside of the regenerator. The heat extractor can be an internal heat extractor (arranged inside the regenerator body) or/and an external heat extractor (arranged outside the regenerator body). The heat extractor is one or more, supplying the excess energy generated by the regenerator to other devices. The excess heat of the regeneration system can be used to generate high-pressure steam through the heat extractor, which is then exported to other devices for energy supply. In a specific embodiment, by providing a heat extractor, the bed temperature of the regenerator is controlled not to exceed 750 °C, for example, not to exceed 720 °C. In the present application, the heat extractor 715 is provided in the regeneration section to extract the heat of the regeneration section bed and transport it to the outside of the regenerator, and control the temperature of the regeneration section bed.

In a specific embodiment, the temperature of the regeneration section bed is controlled by the heat extractor to be no more than 750 °C, preferably no more than 720 °C.

As shown in FIG. 4, the regeneration unit 700 further includes a cyclone separator 720. The regeneration flue gas leaves the regenerator through the cyclone separator 720 and enters the flue gas energy recovery system 730 to recover energy. The cyclone separator 720 may be provided inside the regenerator 740.

The catalyst suitable for the catalyst regeneration method and system of the present application can be a catalyst conventionally used in various catalytic cracking processes, and the present application has no particular restrictions. In some specific embodiments, the catalyst includes zeolite, inorganic oxide and optional clay, and each component accounts for as follows, based on the total weight of the catalyst: zeolite 1% by weight-50% by weight, inorganic oxide 5% by weight-99% by weight, and clay 0% by weight-70% by weight. Wherein zeolite is an active component, selected from medium-pore zeolite and/or optional large-pore zeolite, with medium-pore zeolite accounting for 10% by weight-100% by weight of the total weight of zeolite, and large-pore zeolite accounting for 0% by weight-90% by weight of the total weight of zeolite. Medium-pore zeolite is selected from one or more of ZSM series zeolites and/or ZRP zeolite, and the above zeolite can be modified with non-metals such as phosphorus, etc., and/or transition metals such as iron, cobalt, nickel, etc. Large-pore zeolite is selected from one or more of hydrogen Y, rare earth Y, rare earth hydrogen Y, ultra-stable Y, etc.

### Example

The present application will be further described below referring to examples, but the present application is not limited in any way thereby.

The properties of the feedstock oils A and B used in the following examples and comparative examples are listed in Tables 1 and 2, respectively, and the composition of feedstock C is listed in Table 3.

**Table 1. Properties of feedstock oil A**

| Feed properties | Feedstock oil A |
|---|---|
| Density, kg/m³ (20 °C ) | 890.5 |
| Conradson carbon residue, weight% | 2.94 |
| C, weight% | 86.48 |
| H, weight% | 13.18 |
| S, weight% | 0.15 |
| N, weight% | 0.19 |
| Fe, µg/g | 9.1 |
| Na, µg/g | 0.16 |
| Ni, µg/g | 5.4 |
| V, µg/g | 16.49 |
| Initial boiling point, °C | 261 |
| 10%, °C | 439 |
| 30%, °C | 501 |
| 50%, °C | 550 |

**Table 2. Properties of feedstock oil B**

| Feed properties | Feedstock oil B |
|---|---|
| Density, kg/m³ (20 °C ) | 843.7 |
| C, weight% | 86.59 |
| H, weight% | 13.41 |
| S, µg/g | 5800 |
| N, µg/g | 62 |
| Initial boiling point, °C | 226 |
| 50% distillation temperature, °C | 287 |
| Paraffins, weight% | 40.5 |
| Cycloalkanes, weight% | 33.3 |
| Aromatics, weight% | 26.2 |

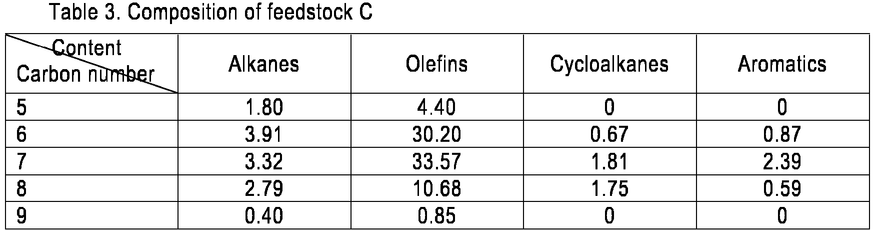

Catalyst a is commercially available catalyst ASC-2, the properties of which are listed in Table 4;

**Table 4. Properties of Catalyst a**

| Catalyst brand | Catalyst a |
|---|---|
| Micro-activity index, % | 47 |
| Specific surface area (m²/g) | 104 |
| Total pore volume, ml/g | 0.153 |

| Metal weight fraction, % | |
|---|---|
| Fe | 0.57 |
| Ni | 1.34 |
| V | 0.33 |
| Sb | 0.35 |

Catalyst b is a TCC catalyst, which was used after aging. The aging conditions were: aging with steam at 800 °C for 15 hours. The preparation process of catalyst b was as follows:
969g of halloysite (product of China Kaolin Company, solid content 73%) was slurried with 4300g of decationized water, and then 781g of pseudo-boehmite (product of Shandong Zibo Bauxite Factory, solid content 64%) and 144ml of hydrochloric acid (concentration 30%, specific gravity 1.56) were added and stirred evenly, and the mixture was allowed to stand and age at 60 °C for 1 hour, keeping pH at 2-4, and then cooled to room temperature, and then 5000g of high silicon-aluminum ratio mesoporous shape-selective ZSM-5 zeolite slurry containing chemical water prepared in advance was added, stirred evenly, spray dried, and free Na⁺ was washed away to obtain the catalyst. The properties are listed in Table 5.

**Table 5. Properties of Catalyst b**

| Catalyst | b |
|---|---|
| Chemical composition/weight% | |
| Al₂O₃ | 49.2 |
| Na₂O | 0.07 |

| Physical properties | |
|---|---|
| Specific surface area/(m²·g⁻¹) | / |
| Bulk density/(g·cm⁻³) | 0.79 |
| Wear index/(%·h⁻¹) | 1.1 |

| Screening composition/weight% | |
|---|---|
| 0-40 µm | 14.2 |
| 0-80 µm | 53.8 |
| 0-149 µm | 89.5 |

The preparation process of catalyst c was as follows:
(1) 20 g of NH₄Cl was dissolved in 1000 g of water, to the solution 100 g (dry basis) of crystallized product ZRP-1 molecular sieve (produced by Qilu Petrochemical Company Catalyst Plant, SiO₂/Al₂O₃ =30, rare earth content RE₂O₃=2.0 wt%) was added, exchanged at 90 °C for 0.5 hour, filtered to obtain filter cake. 4.0 g of H₃PO₄ (concentration 85%) and 4.5 g of Fe(NO₃)₃ dissolved in 90 g of water were added, mixed with the filter cake, impregnated and dried; then calcined at 550 °C for 2 hours to obtain MFI mesoporous molecular sieve containing phosphorus and iron. The elemental analysis chemical composition of the obtained molecular sieve is: 0.1Na₂O•5.1Al₂O₃•2.4P₂O₅•1.5Fe₂O₃•3.8RE₂O₃•88.1SiO₂.
(2) 75.4 kg of halloysite (industrial product of Suzhou Clay Company, solid content 71.6 wt%) was slurried with 250 kg of decationized water, and then 54.8 kg of pseudo-boehmite (industrial product of Youdong Aluminum Plant, solid content 63 wt%) was added. The pH value was adjusted to 2-4 with hydrochloric acid, and the mixture was stirred evenly. The mixture was allowed to stand and age at 60-70 °C for 1 hour, maintaining the pH value at 2-4. The temperature was lowered to below 60 °C, and 41.5 kg of aluminum sol (product of Qilu Petrochemical Company Catalyst Plant, Al₂O₃ content 21.7 wt%) was added. The mixture was stirred for 40 minutes to obtain a mixed slurry.
(3) The MFI mesoporous molecular sieve containing phosphorus and iron (2 kg on a dry basis) prepared in step (1) was added to the mixed slurry obtained in step (2), stirred evenly, spray-dried to form, washed with an ammonium dihydrogen phosphate solution (phosphorus content of 1 wt%) to remove free Na⁺, and dried to obtain a catalytic conversion catalyst c sample. Based on the total dry basis weight of catalyst c, the dry basis composition of catalyst c included: 2wt% of MFI mesoporous molecular sieve containing phosphorus and iron, 36 wt% of pseudo-boehmite and 8 wt% of aluminum sol, and a balance of kaolin.

### Example 1

The test was carried out using the device shown in Fig. 1, in which the specific preparation process of the gaseous fuel was as follows:
The biomass was pretreated in a preprocessor unit by washing with water or acid, and then crushing, grinding, etc. The pretreated biomass was fermented in an anaerobic fermentation device at a fermentation temperature of 37 °C. After the fermentation product was dehydrated by a drying device, a gaseous product was obtained, with methane accounting for more than 40% (based on the volume of the gaseous product), which was stored in a storage tank for standby use.

Feedstock A was used as the reaction feedstock, and catalytic conversion catalyst a was used as the catalyst. The catalyst to be regenerated was regenerated according to the method of the present application. The catalyst to be regenerated from the to-be-regenerated inclined pipe contacted the gaseous fuel introduced from the gas distributor at the bottom of the regenerator and air, and a coke-burning reaction occurred. The excess energy generated by the regeneration system was used to supply energy to outside through the heat extraction system. The operating temperature of the regenerator was 685 °C, the average catalyst residence time was 5 minutes, and the gas superficial linear velocity was 1.0m/s. The regenerated catalyst entered the reactor and contacted with the feedstock oil for catalytic cracking reaction. The excess energy was used to supply other devices through the heat extraction system. The regeneration conditions, reaction conditions, and carbon dioxide emissions are shown in Table 6.

### Comparative Example 1

The test was carried out with reference to Example 1, except that the biomass processing unit was not used, but diesel was injected into the regenerator as fuel oil, and the fuel oil was used as a source of supplementary energy. The regenerator coke-burning temperature was 685 °C. The average catalyst residence time was 5 minutes, and the gas superficial linear velocity was 1.0 m/s. The regenerated catalyst entered the reactor and contacted with the feedstock oil for catalytic cracking reaction. The regeneration conditions, reaction conditions, and carbon dioxide emissions are shown in Table 6.

**Table 6. Operating conditions and results of Example 1 and Comparative Example 1**

| Project | Example 1 | Comparative Example 1 |
|---|---|---|
| Hydrocarbons cracking unit | | |
| Catalyst | a | a |
| Feedstock | Feedstock oil A | Feedstock oil A |
| Reaction temperature, °C | 530 | 530 |
| Reaction time, seconds | 1.5 | 1.5 |
| Steam/feedstock oil weight ratio | 0.1 | 0.1 |
| Catalyst/feedstock oil weight Ratio | 8 | 8 |

| Product distribution, weight% | | |
|---|---|---|
| Dry gas | 4.67 | 4.52 |
| Liquefied gas | 43.66 | 43.75 |
| Propylene | 30.95 | 30.81 |
| Gasoline | 21.41 | 21.39 |
| Diesel | 24.35 | 24.47 |
| Coke | 5.90 | 5.87 |
| Total | 100 | 100 |

| Regeneration unit | | |
|---|---|---|
| Regeneration temperature, °C | 685 | 685 |
| Average catalyst residence time, minutes | 6 | 6 |
| Energy Combustion Medium | Biomass methane-rich gas | Fuel oil |
| Supplement amount^{†} | 1.256×10⁻³ m³ | 1.164 g |

| Regeneration system carbon dioxide emission index^{‡} | | |
|---|---|---|
| gCO₂/MJ | 71.82 | 85.00 |

| | | |
|---|---|---|
| †: Based on processing 100g of feedstock. ‡: The carbon dioxide emission index refers to the amount of carbon dioxide from fossil energy emitted for every 1 MJ of energy generated by the coke-burning in the regeneration system. The calculation method refers to the "Guidelines for Accounting and Reporting Greenhouse Gas Emissions China Petrochemical Enterprises (Trial)" (hereinafter the same); the carbon dioxide produced by biomass methane-rich gas comes from the carbon dioxide present in the atmosphere, which represents a neutral carbon emission process. | | |

From the data in Table 6, it can be seen that when the example uses gaseous biomass fuel as a source of supplementary energy, when the regeneration system produces the same amount of energy, the amount of carbon dioxide emitted is significantly reduced compared with the comparative example, and more energy can be transferred to other devices, which is conducive to fundamentally reducing carbon dioxide emissions.

### Example 2

The test was conducted using the device shown in FIG. 2, in which a flue gas energy recovery system 430 and a carbon dioxide separation system 460 were provided, and the reactor 110 was a conventional riser reactor.

The specific preparation process of gaseous fuel was as follows:
The biomass was pretreated in a preprocessor unit by washing with water or acid, and then crushing, grinding, etc. The pretreated biomass was fermented in an anaerobic fermentation device at a fermentation temperature of 37 °C. After the fermentation product was dehydrated by a drying device, a gaseous product was obtained, with methane accounting for more than 40% (based on the volume of the gaseous product), which was stored in a storage tank for standby use.

Feedstock B was used as the reaction feedstock, and catalytic conversion catalyst b was used as the catalyst. The catalyst to be regenerated was regenerated according to the method of the present application. The gaseous fuel was introduced into the first regenerator through the gas distributor, underwent a combustion reaction together with the catalyst to be regenerated that entered the first regenerator through the to-be-regenerated inclined pipe, and a partial coke-burning reaction occurred in the first regenerator (first stage regeneration); the partially regenerated catalyst was transported to the second regenerator through the catalyst transport pipe to undergo a coke-burning reaction for complete regeneration. At the same time, a part of the flue gas discharged from the regenerator cyclone separation system returned to the first regenerator and the second regenerator, and the oxygen content was controlled not to exceed 28%. The excess energy generated by the regeneration system was used to supply energy to outside through the heat extractor.

The operating temperature of the first regenerator was 650 °C, the average catalyst residence time in the first regenerator was 120 seconds, and the gas superficial linear velocity was 1.5 m/s. The operating temperature of the second regenerator was 670 °C, the average catalyst residence time in the second regenerator was 3.0 minutes, and the gas superficial linear velocity was 1.0 m/s. The regenerated catalyst entered the reactor and contacted with the feedstock oil for catalytic cracking reaction. The regeneration conditions, reaction conditions, and carbon dioxide emissions are shown in Table 7.

### Comparative Example 2

The test was conducted with reference to Example 2, except that diesel was used as the fuel oil, which was injected into the to-be-regenerated pipeline and premixed with the catalyst to be regenerated before entering the regenerator as a source of supplementary energy. The regeneration conditions, reaction conditions, and carbon dioxide emissions are shown in Table 7.

**Table 7. Operating conditions and results of Example 2 and Comparative Example 2**

| Project | Example 2 | Comparative Example 2 |
|---|---|---|
| Hydrocarbons cracking unit | | |
| Catalyst | b | b |
| Feedstock | Feedstock B | Feedstock B |
| Reaction temperature, °C | 530 | 530 |
| Catalyst to oil ratio | 8.0 | 8.0 |
| Space velocity, h⁻¹ | 10 | 10 |

| Product distribution, weight% | | |
|---|---|---|
| Dry gas | 1.27 | 1.55 |
| Liquefied gas | 11.97 | 10.25 |
| Propylene | 6.03 | 4.79 |
| Gasoline | 14.02 | 14.96 |
| Diesel | 70.02 | 68.97 |
| Catalytic cracking distillate | 2.40 | 3.87 |
| Coke | 0.32 | 0.40 |
| Total | 100 | 100 |

| Regeneration unit | | |
|---|---|---|
| First regenerator temperature, °C | 650 | 650 |
| Average residence time in the first regenerator, seconds | 120 | 120 |
| Second regenerator temperature, °C | 670 | 670 |
| Average residence time in the second regenerator, minutes | 3.0 | 3.0 |
| Supplemental energy combustion medium | Biomass methane-rich gas | Fuel oil |
| Supplement Amount^{†} | 7.515×10⁻³ m³ | 6.887 g |

| Regeneration system carbon dioxide emission index^{‡} | | |
|---|---|---|
| gCO₂/MJ | 3.41 | 71.79 |

| | | |
|---|---|---|
| †: Based on processing 100g of feedstock. ‡: The carbon dioxide emission index refers to the amount of carbon dioxide from fossil energy emitted for every 1 MJ of energy generated by the coke-burning in the regeneration system; the carbon dioxide produced by the gaseous fuel comes from the carbon dioxide present in the atmosphere, which represents a neutral carbon emission process. | | |

From the data in Table 7, it can be observed that when the example uses the gaseous fuel obtained by biomass gasification as a source of supplementary energy, when the regeneration system produces the same amount of energy, the amount of carbon dioxide emitted is significantly reduced compared with the comparative example, which is conducive to fundamentally reducing the emission of carbon dioxide; on the other hand, the yield of propylene in Example 2 is also improved to a certain extent, indicating that the selectivity of the regenerated catalyst for propylene is improved.

### Example 3

The test was conducted using the device shown in FIG. 3, in which a flue gas energy recovery system 530 and a carbon dioxide separation system 560 were provided, and the reactor 110 was a conventional riser reactor.

The biomass processing unit 600 included: a biomass preprocessor 610, a biomass gasifier 620, and a gaseous fuel storage tank 630. The operating conditions of the biomass gasifier 620 were: a temperature of 950 °C, a gasification medium of steam, and the volume fractions of hydrogen, carbon monoxide, and methane in the obtained gaseous product were 50%, 25%, and 7%, respectively, based on the volume of the gaseous product.

Feedstock C was used as the reaction feedstock, and catalytic conversion catalyst c was used as the catalyst. The catalyst to be regenerated was regenerated according to the method of the present application. Pure oxygen gas was introduced into the first regenerator and the second regenerator respectively, and the gaseous fuel from the gaseous fuel storage tank 630 was introduced from the gas distributor of the first regenerator, underwent a combustion reaction together with the catalyst to be regenerated from the inclined pipe. At the same time, a part of the flue gas discharged from the regenerator cyclone separation system 520 was returned to the bottom of the first regenerator and the second regenerator, and the oxygen content was controlled not to exceed 28%. The excess energy generated by the regeneration system was used to supply energy to outside through the heat extractor.

The operating temperature of the first regenerator was 630 °C, the average catalyst residence time in the first regenerator was 40 seconds, and the gas superficial linear velocity was 1.0 m/s. The operating temperature of the second regenerator was 665 °C, the average catalyst residence time in the second regenerator was 1.5 minutes, and the gas superficial linear velocity was 1.0 m/s. The regenerated catalyst entered the reactor and contacted with the feedstock oil for catalytic cracking reaction. The regeneration conditions, reaction conditions, and carbon dioxide emissions are shown in Table 8.

### Comparative Example 3

The test was conducted with reference to Example 3, except that diesel was used as the fuel oil, which was injected into the to-be-regenerated pipeline and premixed with the catalyst to be regenerated before entering the regenerator as a source of supplementary energy. The regeneration conditions, reaction conditions, and carbon dioxide emissions are shown in Table 8.

**Table 8. Operating conditions and results of Example 3 and Comparative Example 3**

| Project | Example 3 | Comparative Example 3 |
|---|---|---|
| Hydrocarbons cracking unit | | |
| Catalyst | c | c |
| Feedstock | Feedstock C | Feedstock C |
| Reaction temperature, °C | 680 | 680 |
| Reaction time, seconds | 10 | 10 |
| Catalyst to oil ratio | 30 | 30 |

| Product distribution, weight% | | |
|---|---|---|
| Dry gas | 28.21 | 28.25 |
| Liquefied gas | 47.01 | 46.90 |
| Propylene | 30.08 | 29.95 |
| BTX | 6.45 | 6.63 |
| Light oil | 17.93 | 17.79 |
| Coke | 0.40 | 0.43 |
| Total | 100 | 100 |
| Ethylene + Propylene + Butene | 64.64 | 64.31 |

| Regeneration unit | | |
|---|---|---|
| First regenerator temperature, °C | 630 | 630 |
| Average residence time in the first regenerator, seconds | 40 | 40 |
| Second regenerator temperature, °C | 665 | 665 |
| Average residence time in the second regenerator, minutes | 1.5 | 1.5 |
| Supplemental energy combustion medium | Biomass Synqas | Fuel oil |
| Supplement Amount^{†} | 2.593×10⁻² m³ | 11.307 g |

| Regeneration system carbon dioxide emission index^{‡} | | |
|---|---|---|
| gCO₂/MJ | 2.88 | 78.00 |

| | | |
|---|---|---|
| †: Based on processing 100g of feedstock. ‡: The carbon dioxide emission index refers to the amount of carbon dioxide from fossil energy emitted for every 1 MJ of energy generated by the coke-burning in the regeneration system; the carbon dioxide produced by the gaseous fuel comes from the carbon dioxide present in the atmosphere, which represents a neutral carbon emission process. | | |

From the data in Table 8, it can be observed that when the example uses gaseous fuel as a source of supplementary energy and when the regeneration system produces the same amount of energy, the amount of carbon dioxide emitted is significantly reduced compared with the comparative example, which is conducive to fundamentally reducing the emission of carbon dioxide.

### Example 4

The regeneration was performed using the device shown in FIG. 4, in which a flue gas energy recovery system 730 and a carbon dioxide separation system 760 were provided, and the reactor 110 was a conventional riser reactor.

The biomass processing unit 600 included: a biomass preprocessor 610, a biomass gasifier 620, and a gaseous fuel storage tank 630. The operating conditions of the biomass gasifier 620 were: a temperature of 950 °C, a gasification medium of steam, and the volume fractions of hydrogen, carbon monoxide, and methane in the obtained gaseous product were 50%, 25%, and 7%, respectively, based on the volume of the gaseous product.

Feedstock B was used as the reaction feedstock, and catalytic conversion catalyst b was used as the catalyst. The catalyst to be regenerated was regenerated according to the method of the present application. Pure oxygen gas was introduced into the coke-burning section and the regeneration section respectively, and the gaseous fuel from the gaseous fuel storage tank 630 was introduced from the gas distributor at the bottom of the coke-burning section to undergoes a combustion reaction with the catalyst to be regenerated from the inclined pipe. At the same time, a part of the flue gas discharged from the regenerator cyclone separation system 720 was returned to the bottom of the coke-burning section and the regeneration section, and the oxygen content was controlled not to exceed 28%. The excess energy generated by the regeneration system was used to supply energy to outside through the heat extractor.

The operating temperature of the coke-burning section was 630 °C, the average catalyst residence time in the coke-burning section was 40 seconds, and the gas superficial linear velocity was 1.2 m/s. The operating temperature of the dense phase regeneration section was 670 °C, the average catalyst residence time in the dense phase regeneration section was 1.5 minutes, and the gas superficial linear velocity was 0.8 m/s. The regenerated catalyst entered the reactor and contacted with the feedstock oil for catalytic cracking reaction. The regeneration conditions, reaction conditions, and carbon dioxide emissions are shown in Table 9.

### Comparative Example 4

The test was conducted with reference to Example 4, except that diesel was used as the fuel oil, which was injected into the to-be-regenerated pipeline and premixed with the catalyst to be regenerated before entering the regenerator as a source of supplementary energy. The regeneration conditions, reaction conditions, and carbon dioxide emissions are shown in Table 9.

**Table 9. Operating conditions and results of Example 4 and Comparative Example 4**

| Project | Example 4 | Comparative Example 4 |
|---|---|---|
| Hydrocarbons cracking unit | | |
| Catalyst | b | b |
| Feedstock | Feedstock B | Feedstock B |
| Reaction temperature, °C | 530 | 530 |
| Catalyst to oil ratio | 8.0 | 8.0 |
| Space velocity, h⁻¹ | 10 | 10 |
| Product distribution, weight% | | |
| Dry gas | 1.34 | 1.38 |
| Liquefied gas | 10.27 | 10.10 |
| Propylene | 4.79 | 4.70 |
| Gasoline | 14.93 | 14.69 |
| Diesel | 69.37 | 69.73 |
| Catalytic cracking distillate | 3.66 | 3.70 |
| Coke | 0.42 | 0.41 |
| Total | 100 | 100 |

| Regeneration unit | | |
|---|---|---|
| Coke-burning section temperature, °C | 630 | 630 |
| Average residence time in the coke-burning section, seconds | 40 | 40 |
| Regeneration section temperature, °C | 670 | 670 |
| Average residence time in the regeneration section, minutes | 1.5 | 1.5 |
| Supplemental energy combustion medium | Biomass Syngas | Fuel oil |
| Supplement Amount^{†} | 1.482×10⁻² m³ | 6.461 g |

| Regeneration system carbon dioxide emission index^{‡} | | |
|---|---|---|
| gCO₂/MJ | 5.15 | 78.28 |

| | | |
|---|---|---|
| †: Based on processing 100g of feedstock. ‡: The carbon dioxide emission index refers to the amount of carbon dioxide from fossil energy emitted for every 1 MJ of energy generated by the coke-burning in the regeneration system; the carbon dioxide produced by the gaseous fuel comes from the carbon dioxide present in the atmosphere, which represents a neutral carbon emission process. | | |

From the data in Table 9, it can be observed that when the example uses gaseous fuel obtained by biomass gasification as a source of supplementary energy and when the regeneration system produces the same amount of energy, the amount of carbon dioxide emitted is significantly reduced compared with the comparative example, which is conducive to fundamentally reducing the emission of carbon dioxide.

The preferred embodiments of the present application are described in details above; however, the present application is not limited to the specific details in the above embodiments. Within the technical concept of the present application, a variety of simple modifications can be made to the technical solution of the present application, and these simple modifications all fall within the protection scope of the present application.

It should also be noted that the various specific technical features described in the above specific embodiments can be combined in any suitable manner as long as they don't conflict. In order to avoid unnecessary repetition, this application does not further describe various possible combinations.

In addition, the various embodiments of the present application may be arbitrarily combined, as long as they do not violate the concept of the present application, and they should also be regarded as the contents disclosed in the present application.

## Claims

1. A catalyst regeneration method suitable for a fluidized catalytic cracking unit comprising a catalytic cracking reactor and a catalyst regenerator, wherein the regeneration method comprises the following steps:
1) providing a gaseous biomass-derived fuel containing hydrogen and/or methane, obtained by, for example, gasification or anaerobic fermentation of biomass;
2) directly feeding the gaseous fuel into the catalyst regenerator without separation and purification;
3) introducing an oxygen-containing gas into the catalyst regenerator, wherein the oxygen content of the oxygen-containing gas is 14-28% by volume; and
4) feeding the catalyst to be regenerated from the catalytic cracking reactor into the catalyst regenerator, where it contacts with the gaseous fuel and the oxygen-containing gas for coke-burning and regeneration,
preferably, the operating temperature of the catalyst regenerator is in the range of 550-750 °C, and the average catalyst residence time is 1.0-15.0 minutes.

2. The method according to claim 1, wherein the gaseous fuel is injected into the catalyst regenerator through a gas distributor from a position not lower than the level of the catalyst to be regenerated inlet, and the oxygen-containing gas is air or is oxygen diluted with recycled flue gas;
preferably, when the oxygen-containing gas is air, the amount of the gaseous fuel introduced is no more than 13% by volume of the amount of air introduced, preferably 3-13% by volume, or when the oxygen-containing gas is oxygen diluted by recycled flue gas, the amount of the gaseous fuel introduced is no more than 44% by volume, preferably 10-44 % by volume of the amount of oxygen introduced.

3. The method according to claim 1 or 2, wherein the catalyst regenerator is a single-stage regenerator, and the operating conditions of the regenerator include: an operating temperature of 550-750 °C, an average catalyst residence time of 1.0-15.0 minutes, and a gas superficial linear velocity of 0.5-2.0 m/s.

4. The method according to claim 1 or 2, wherein the catalyst regenerator is a two-stage regenerator comprising a coke-burning section and a regeneration section which are in fluid communication, and in step 2), the gaseous fuel is fed into the coke-burning section and/or the regeneration section, preferably only into the coke-burning section, in step 3), the oxygen-containing gas is introduced into the bottom of the coke-burning section and the regeneration section respectively, and in step 4), the catalyst to be regenerated is fed into the coke-burning section,
preferably, the operating conditions of the coke-burning section include: an operating temperature of 550-720 °C, an average catalyst residence time of 10.0-120.0 seconds, preferably 15.0-90.0 seconds, and a gas superficial linear velocity of 0.5-5.0 m/s, preferably 1.0-4.0 m/s; and
the operating conditions of the regeneration section include: an operating temperature of 600-750 °C, an average catalyst residence time of 0.5-5.0 minutes, preferably 1.0-4.0 minutes, and a gas superficial linear velocity of 0.4-2.0 m/s, preferably 0.5-1.5 m/s;
further preferably, the operating temperature of the regeneration section is 10-150 °C higher than the operating temperature of the coke-burning section.

5. The method according to claim 1 or 2, wherein the catalyst regenerator is a dual regenerator comprising a first regenerator and a second regenerator which are in fluid communication, and in step 2), the gaseous fuel is fed into the first regenerator and/or the second regenerator, preferably only into the first regenerator, in step 3), the oxygen-containing gas is introduced into the bottom of the first regenerator and the second regenerator, respectively, and in step 4), the catalyst to be regenerated is fed into the first regenerator,
preferably, the operating conditions of the first regenerator include: an operating temperature of 550-720 °C, an average catalyst residence time of 20.0-240.0 seconds, preferably 30.0-150.0 seconds, and a gas superficial linear velocity of 0.5-5.0 m/s, preferably 1.0-4.0 m/s; and
the operating conditions of the second regenerator include: an operating temperature of 600-750 °C, an average catalyst residence time of 0.5-5.0 minutes, preferably 1.0-4.0 minutes, and a gas superficial linear velocity of 0.4-2.0 m/s, preferably 0.5-1.5 m/s;
further preferably, the operating temperature of the second regenerator is 10-150 °C higher than the operating temperature of the first regenerator.

6. The method according to claim 4 or 5, wherein the coke-burning ratio in the coke-burning section or the first regenerator is 40-70%, preferably 40-50%; and the coke-burning ratio in the regeneration section or the second regenerator is 30-60%, preferably 50-60%.

7. The method according to any one of claims 1 to 6, wherein the gaseous fuel is obtained by gasification of biomass and comprises, based on the total volume of the gaseous fuel, 12-60% of hydrogen, 15-30% of carbon monoxide and 3-8% of methane, and the remainder is carbon dioxide and/or nitrogen; or
the gaseous fuel is obtained through anaerobic fermentation of biomass and comprises 40-100 % by volume of methane based on the total volume of the gaseous fuel.

8. The method according to any one of claims 1 to 7, wherein step 1) further comprises:
gasifying the biomass in the presence of a gasification medium at a gasification temperature of 500-1500 °C, wherein the gasification medium is selected from air, oxygen/oxygen-enriched gas, and steam; or
subjecting the biomass to anaerobic fermentation in a closed fermentation tank, and the fermentation temperature is not higher than 60 °C.

9. The method according to claim 8, wherein the biomass is pretreated before gasification or anaerobic fermentation, and the pretreatment is selected from one or more of grinding, drying, extrusion, steam explosion, acid treatment, alkali treatment and microbial pretreatment.

10. A catalyst regeneration system suitable for a fluidized catalytic cracking unit, comprising a biomass processing unit and a catalyst regeneration unit, wherein:
the biomass processing unit is used to process biomass, for example by gasification or anaerobic fermentation, to obtain a gaseous fuel containing hydrogen and/or methane, and comprises a gaseous fuel generator and a gaseous fuel storage tank, wherein the gaseous fuel generator is preferably selected from a biomass gasifier, a biomass anaerobic fermentation tank or a combination thereof, and has a biomass inlet and a gaseous product outlet, wherein the gaseous fuel storage tank has an inlet and a gaseous fuel outlet, wherein the gaseous product outlet of the gaseous fuel generator is connected to the inlet of the gaseous fuel storage tank;
wherein the catalyst regeneration unit is used to regenerate the catalyst to be regenerated from the catalytic cracking reactor, and comprises a catalyst regenerator, wherein the catalyst regenerator has a catalyst to be regenerated inlet, an oxygen-containing gas inlet, a gaseous fuel inlet, a regeneration flue gas outlet, and a regenerated catalyst outlet, and
wherein the gaseous fuel outlet of the gaseous fuel storage tank is connected to the gaseous fuel inlet of the catalyst regenerator through a pipeline.

11. The catalyst regeneration system according to claim 10, wherein the biomass processing unit further comprises a biomass preprocessor and an optional gaseous product dryer, wherein the biomass preprocessor is used to pretreat the biomass, wherein the pretreatment is selected from one or more of grinding, drying, extrusion, steam explosion, acid treatment, alkali treatment and microbial pretreatment, and the gaseous product dryer is used to dry the gaseous product obtained from the biomass anaerobic fermentation tank.

12. The catalyst regeneration system according to claim 10 or 11, wherein the catalyst regenerator comprises a coke-burning section and a dense phase regeneration section, the dense phase regeneration section being located above the coke-burning section, and wherein the outlet of the coke-burning section is accommodated inside the dense phase regeneration section, so that the coke-burning section is in fluid communication with the dense phase regeneration section;
wherein the coke-burning section is provided with:
a first oxygen-containing gas inlet, which is provided at the bottom of the coke-burning section and is used to feed an oxygen-containing gas into the coke-burning section;
the gaseous fuel inlet, which is provided above the first oxygen-containing gas inlet and is used to feed the gaseous fuel;
a gas distributor configured to distribute the gaseous fuel fed through the gaseous fuel inlet;
the catalyst to be regenerated inlet, which is used to transport the catalyst to be regenerated from the catalytic cracking reactor to the interior of the coke-burning section; and
an optional first recycled flue gas inlet, which is used to recycle a portion of the flue gas discharged from the dense phase regeneration section back into the interior of the coke-burning section;
wherein the dense phase regeneration section is provided with:
a second oxygen-containing gas inlet, which is provided at the bottom of the dense phase regeneration section and is used to feed an oxygen-containing gas into the dense phase regeneration section;
an optional second gaseous fuel inlet, which is provided above the second oxygen-containing gas inlet and is used to feed the gaseous fuel into the dense phase regeneration section;
an optional second gas distributor configured to distribute the gaseous fuel fed through the second gaseous fuel inlet;
the regeneration flue gas outlet, which is provided at the top of the dense phase regeneration section and is used to discharge the regeneration flue gas in the dense phase regeneration section;
the regenerated catalyst outlet, which is used to return the regenerated catalyst to the catalytic cracking reactor; and
an optional second recycled flue gas inlet, which is used to recycle a portion of the flue gas discharged from the dense phase regeneration section back into the dense phase regeneration section;
optionally, the dense phase regeneration section is further provided with a heat extractor for transferring heat to the outside of the regenerator.

13. The catalyst regeneration system according to claim 10 or 11, wherein the catalyst regenerator comprises a first regenerator and a second regenerator, wherein the second regenerator is located downstream of the first regenerator, wherein the first regenerator and the second regenerator are connected by a catalyst transport pipe to transport the catalyst material partially regenerated by the first regenerator to the second regenerator;
wherein the first regenerator is provided with:
a first oxygen-containing gas inlet, which is provided at the bottom of the first regenerator and is used to feed an oxygen-containing gas into the first regenerator;
the gaseous fuel inlet, which is provided above the first oxygen-containing gas inlet and is used to feed the gaseous fuel;
a gas distributor configured to distribute the gaseous fuel fed through the gaseous fuel inlet;
the catalyst to be regenerated inlet, which is used to transport the catalyst to be regenerated from the catalytic cracking reactor to the interior of the first regenerator;
a first regeneration flue gas outlet, which is provided at the top of the first regenerator and is used to discharge the regeneration flue gas in the first regenerator; and
an optional first recycled flue gas inlet, which is provided at the bottom of the first regenerator and in communication with the first regeneration flue gas outlet, for recycling a portion of the flue gas discharged from the first regenerator back to the first regenerator,
wherein the second regenerator is provided with:
a second oxygen-containing gas inlet, which is provided at the bottom of the second regenerator and is used to feed an oxygen-containing gas into the second regenerator;
an optional second gaseous fuel inlet, which is provided above the second oxygen-containing gas inlet and is used to feed the gaseous fuel into the second regenerator;
an optional second gas distributor configured to distribute the gaseous fuel fed through the second gaseous fuel inlet;
the regenerated catalyst outlet, which is used to return the regenerated catalyst to the catalytic cracking reactor;
a second regeneration flue gas outlet, which is provided at the top of the second regenerator and is used to discharge the regeneration flue gas in the second regenerator; and
an optional second recycled flue gas inlet, which is provided at the bottom of the second regenerator and is connected to the first regeneration flue gas outlet and/or the second regeneration flue gas outlet, for recycling a portion of the flue gas back to the second regenerator.
